# EUROPÄISCHE PATENTANMELDUNG

(11) **EP 0 626 576 A1**
(43) Veröffentlichungstag der Anmeldung: **30.11.1994**
(21) Anmeldenummer: 94104384.6
(22) Anmeldetag: 21.03.1994
(51) Int. Cl.: G01N 21/89

(54) **Verfahren zur berührungslosen optischen Messung von qualitätsbestimmenden Parametern textiler Oberflächen sowie Anordnung zur Durchführung des Verfahrens**

(30) Priorität: 16.04.1993 DE 4312452
(71) Anmelder: ERHARDT + LEIMER GmbH, D-86157 Augsburg (DE)
(72) Erfinder: Massen, Robert Prof. Dr.-Ing.,, D-78337 Öhningen-Wangen (DE); Bräu, Jürgen, D-86153 Augsburg (DE); Wollenweber, Wolf, D-86356 Neusäss (DE); Büchner, Thomas, D-86459 Gesserthausen (DE)
(74) Vertreter: Fay, Hermann, Dipl.-Phys. Dr.

(57) **Zusammenfassung**

Es wird ein multi-funktionales Verfahren zur optischen Bestimmung zahlreicher qualitäts-bestimmender Parameter von textilen Oberflächen mit Hilfe einer gleichen Anordnung von bildgebendem Sensor und besonders ausgebildeter Beleuchtungseinrichtung beschrieben. Die Beleuchtungseinrichtung besteht aus einer Vielzahl von punktförmigen Leuchtelementen, insbesondere Halbleiter-Leuchtioden, welche in einer räumlich gekrümmten, in Zonen eingeteilten Anordnung so angebracht sind, daß sie die textile Oberfläche aus verschiedenen Richtungen und/oder in verschiedenen Bereichen beleuchtet. Für das jeweilig zu messende Qualitätsmerkmal wird die jeweils optimale Beleuchtungskonfiguration elektronisch bestimmt und mit ebenfalls abgestimmten Verfahren der Bildverarbeitung dieses Merkmal aus den abgespeicherten Bildern ermittelt.

## Beschreibung

Die Erfindung betrifft ein Verfahren zur berührungslosen optischen Messung von qualitätsbestimmenden Parametern textiler Oberflächen mit Hilfe bildgebender Sensoren sowie eine Anordnung zur Durchführung des Verfahrens.

Der Einsatz von bildgebenden Sensoren wie Laserabtaster oder Zeilenkamerasysteme wird derzeit bei der Herstellung von Bekleidungstextilien und von technischen Textilien vor allem in zwei Bereichen eingesetzt:
a) Zur Bandkantenregelung an laufenden textilen Bahnen wird die Bahnkante oder ein Muster mit Zeilenkameras beobachtet und aus dem Kamerasignal werden zur Bahnsteuerung Stellsignale gewonnen.
b) Zur automatischen Warenschau wird die textile Oberfläche mit Laserscannern, Zeilenkameras oder Matrixkameras abgetastet und daraus werden mit Verfahren der digitalen Bildverarbeitung lokale Fehler wie Löcher, fehlende Kett/Schußfäden o. ä. automatisch erkannt.

Daneben ist aus dem europäischen Patent 0 160 895 ein Verfahren zur Schrumpfmessung an periodisch strukturierten textilen Oberflächen bekannt, bei welchem mit Hilfe eines bildgebenden Sensors eine Nahaufnahme der Oberfläche erfaßt und aus dem digitalisierten Bild dieses Ausschnittes mit Methoden der Korrelationsanalyse die Schrumpfung der textilen Ware bestimmt wird.

Neben der Schrumpfung gibt es aber eine ganze Reihe von wichtigen textilen Qualitätsmerkmalen, welche für den Produktionsingenieur wichtig sind:
1. Die Anzahl von Maschen und Stäbchen pro Längeneinheit
2. Die Anzahl von Ketten und Schußfäden bei Webware
3. Die Ausrichtung der Maschen und Stäbchen
4. Die Ausrichtung der Kette und der Schußfäden
5. Das Flächengewicht
6. Die lokale Homogenität bei Maschen- und Webware
7. Die Faserigkeit der textilen Oberfläche
8. Das optische Reflexionsverhalten bei verschiedenen Wellenlängen
9. Das optische Transmissionsverhalten bei verschiedenen Wellenlängen
10. Die automatische Identifikation einer Partie
11. Die Konsistenz der visuellen Eigenschaften der Oberfläche
Für einige dieser Merkmale gibt es pysikalisch/mechanische Meßeinrichtungen, welche aber mit erheblichen Nachteilen versehen sind.

So wird derzeit das Flächengewicht durch die Messung der Abschwächung einer Strahlungsquelle, üblicherweise eines Gammastrahlers ermittelt. Solche Verfahren sind vom Strahlenschutz und der späteren Entsorgung her gesehen sehr schwierige und in naher Zunkunft verbotene Verfahren.

Ein optomechanisches Verfahren für die Messung der Ausrichtung der Ketten/Schußfäden oder der Maschen/Stäbchen ist aus der Praxis bekannt. Hierbei wird über eine rotierende Blende die textile Oberfläche abgetastet und aus dem reflektierten Licht die Ausrichtung bestimmt. Der sehr spezielle Meßkopf enthält mechanisch bewegliche Präzisionsteile und ist entsprechend aufwendig in der Herstellung. Mit diesem Gerät kann nur die Ausrichtung gemessen werden.

Es besteht daher ein großes wirtschaftliches und technisches Interesse, mit Hilfe einer einfachen Meßanordnung ohne mechanisch bewegte Teile möglichst viele der o. g. Qualitätsmerkmale gleichzeitig, kontinuierlich, berührungslos und direkt im Prozeß zu messen.

Dies ist mit den derzeit bekannten optischen Verfahren einschließlich derjenigen, welche Matrix- und/oder Zeilenkameras verwenden, nicht möglich, da diese Geräte durch ihren Aufbau, die eingesetzte Beleuchtung und die eingesetzten Signalverarbeitungsverfahren speziell auf eine einzige Meßart ausgelegt sind.

Erfindungsgemäß wird die gestellte Aufgabe bei einem Verfahren der eingangs genannten Art Aufgabe dadurch gelöst, daß die textile Oberfläche ausschnittweise mit einem bildgebenden Sensor erfaßt wird, daß dieser Ausschnitt durch eine Beleuchtungsquelle mit einer steuerbaren Verteilung der dreidimensionalen Beleuchtungsrichtung und/oder der Beleuchtungszeit und/oder der spektralen Verteilung beleuchtet wird, wobei in Abhängigkeit der textilen Oberfläche und der durchzuführenden Meßart die jeweils beste Beleuchtungsart eingestellt wird, daß das Signal des bildgebenden Sensors digitalisiert und in einen oder mehrere Bildspeicher abgelegt wird, und daß mit einer Recheneinheit anhand des oder der abgespeicherten Bilder mit einem jeweils auf die interessierende Meßart abgestimmten Verfahren der digitalen Signal- oder Bildverarbeitung und Mustererkennung alle oder eine Teilmenge folgender qualitätsbestimmender Parameter ermittelt werden:
1. Die Anzahl von Maschen und Stäbchen pro Längeneinheit
2. Die Anzahl von Ketten und Schußfäden bei Webware
3. Die Ausrichtung der Maschen und Stäbchen
4. Die Ausrichtung der Kette und der Schußfäden
5. Das Flächengewicht
6. Die lokale Homogenität bei Maschen- und Webware
7. Die Faserigkeit der textilen Oberfläche
8. Das optische Reflexionsverhalten bei verschiedenen Wellenlängen
10. Die automatische Identifikation einer Partie
11. Die Konsistenz der visuellen Eigenschaften der Oberfläche,
und daß diese Qualitätsmerkmale in einem Qualitätsprotokoll festgehalten werden und/oder als elektrische Stellsignale zur Steuerung des Produktionsprozesses ausgegeben werden.

Weitere Merkmale der Erfindung ergeben sich aus den Ansprüchen und der folgenden Beschreibung von Ausführungsbeispielen.

Das Verfahren sei an einigen einfachen Beispielen exemplarisch, aber nicht einschränkend erklärt. Hierbei wird auf folgende Abbildungen Bezug genommen:
- Fig. 1: zeigt die jeweils für die Webware und die Maschenware beste Anordnung von Kamera und Beleuchtung,
- Fig. 2: zeigt eine räumlich angeordnete Beleuchtungsquelle zur Durchführung des Verfahrens,
- Fig. 3: zeigt ein Beispiel für die gleichzeitige Messung von drei qualitätsbestimmenden Merkmalen.
- Fig. 4: zeigt ein Beispiel einer Beleuchtungseinrichtung für streifenden Lichteinfall
Textile Oberflächen lassen sich in folgende Kategorien unterteilen:
1. Periodisch strukturierte Textilien wie Webware und Maschenware
2. Textilien ohne periodische Oberflächen wie Flies, Filz, Frottee usw.

Eine weitere, beim Einsatz von bildgebenden Sensoren wie Matrix- und Zeilenkamera wichtige Unterscheidung der Textilien ist die Ausgestaltung der dritten Dimension: während Webwaren in der Regel flache, gut auszuleuchtende Gebilde darstellen, sind Maschenwaren durch die Bildung der Maschen und Stäbchen eher dreidimensionale Gebilde, welche bei üblicher Auflichtbeleuchtung nur wenig Kontrast zwischen "Berg" und "Tal" zeigen.

Eine weitere, beim Einsatz bildgebender Sensoren wichtige Unterscheidung ist die in bedruckte und uni-farbige textile Oberflächen. Von einem möglichst allgemein einsetzbaren Meßgerät wird man erwarten, daß es die genannten Messungen möglichst unabhängig von einem bestehenden Druckmuster durchführen kann.

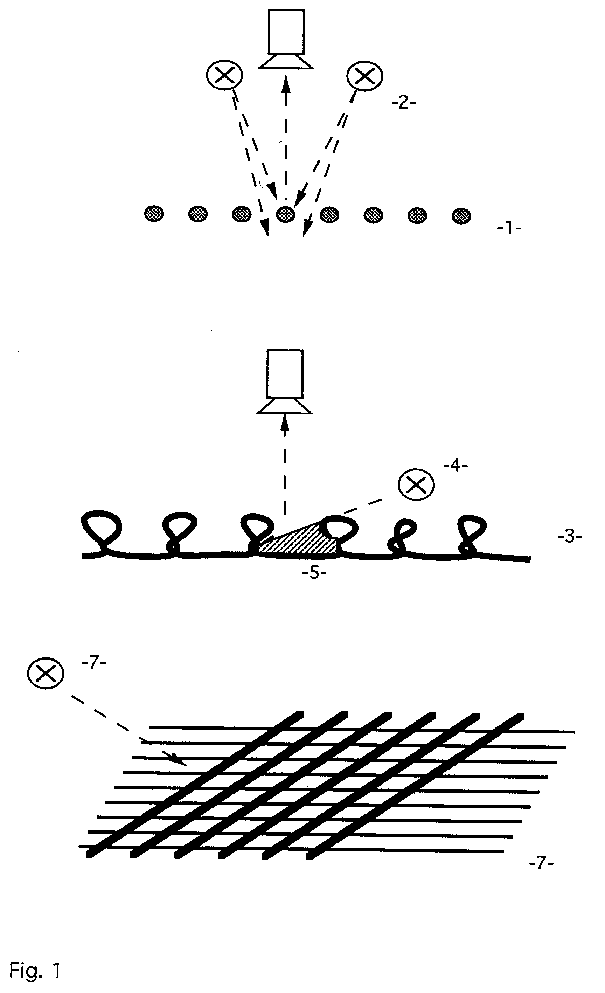

Fig. 1 zeigt den entscheidenden Einfluß der Beleuchtung zur Gewinnung kontrastreicher Bilder bei Webware und bei Maschenware. Webware 1 zeigt eine flache Oberfläche. In den Lücken zwischen Kette und Schuß ist die Relexion gering. Eine solche Oberfläche wird daher optimal durch eine Beleuchtungsquelle 2 beleuchtet, welche in einem kleinen Winkel zur Senkrechten angebracht ist. Maschenware 3 besteht aus einer Reihe von Maschen und Stäbchen, welche eine ausgesprochene "Gebirgslandschaft" bilden. Die Erkennung wird besonders gut, wenn durch eine Beleuchtung 4 im großen Winkel zur Senkrechten beleuchtet wird, weil dann durch die geworfenen Schatten 5 ein besonders kontrastreiches Bild entsteht.

Bei besonderen Bindungsarten wie z.B. der Köper-Bindung 6 wird die beste Beleuchtungsrichtung 7 so gewählt werden, daß sie senkrecht zu der diagonal verlaufenden Struktur verläuft.

Je nach Farbe der verarbeiteten Fäden kann die Sichtbarkeit der Ketten- und Schußfäden bzw. der Maschen und Stäbchen durch eine entsprechende spektrale Verteilung der Beleuchtung optimiert werden. So ist insbesondere eine Beleuchtung im nahen Infrarot (NIR) gut geeignet, um einen von der Farbe und von einem evtl. aufgebrachten Druck unabhängigen Kontrast zu erhalten. Bei der bildgebenden Erfassung einer z. B. grünen textilen Oberfläche empfiehlt es sich, die eingebrachte Beleuchtungsenergie nur in diesem Bereich zu konzentrieren, da alle anderen Wellenlängenbereiche absorbiert und damit für die Aussteuerung des bildgebenden Sensors verloren gehen.

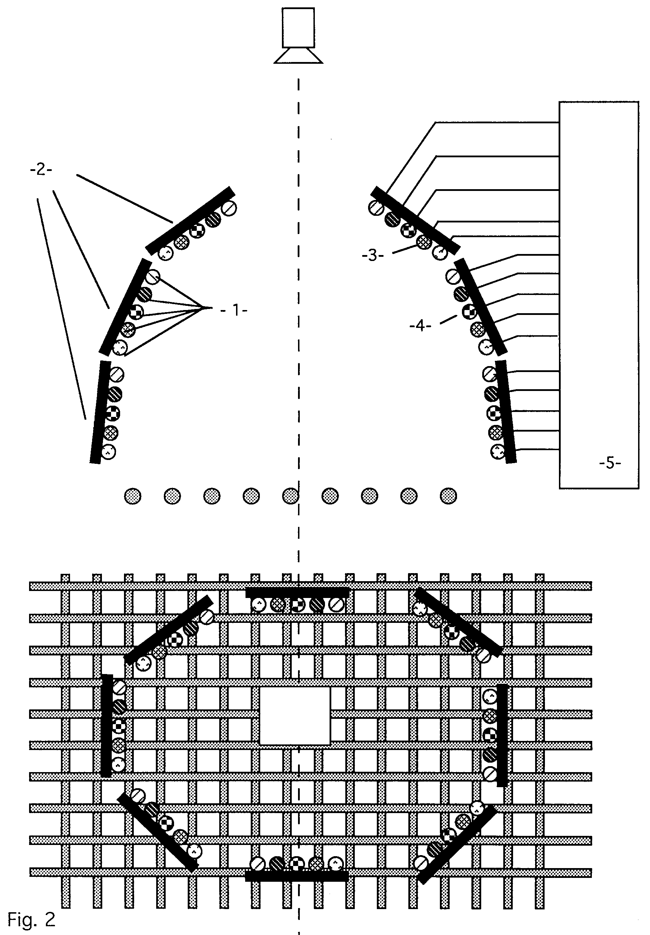

Erfindungsgemäß wird daher eine Beleuchtung nach Fig. 2 eingesetzt, welche aus einer Vielzahl von kleinen Leuchtelementen 1 besteht, die zu verschiedenen Zonen 2 zusammengefaßt sind, wobei sich diese Zonen durch ihre räumliche Anordnung in Bezug zur Oberflächennormalen und zur Ausrichtung relativ zu Ketten/Schußfäden - bzw. Maschen/Stäbchen unterscheiden. Jede Zone enthält Leuchtelemente unterschiedlichen Spektrums, insbesondere solche mit einer vorgegebenen Farbe 3 und solche, welche im nahen Infrarotbereich 4 emittieren. Mit Hilfe einer rechnersteuerbaren Auswahlschaltung 5 können bestimmte Zonen und pro Zone bestimmte Leuchtelemente ausgewählt werden. Durch Steuerung der Belichtungszeit jedes Leuchtelements kann die Beleuchtungsdauer der Empfindlichkeit des bildgebenden Sensors angepaßt werden. Durch verkürzte, stroboskopische Beleuchtung können auch bei schnell bewegten textilen Oberflächen scharfe Bilder erreicht werden. Durch die Aufnahme einer Sequenz von Einzelbildern mit jeweils unterschiedlicher Beleuchtung in punkto Ort, Farbe und Zeit können aus dem Ausschnitt einer textilen Oberfläche mehrere Bilder kurzzeitig nacheinander gewonnen werden, welche jeweils für einen oder eine Gruppe der zu bestimmenden Qualitätsparameter optimal ausgeleuchtet sind.

Erfindungsgemäß bestehen die Leuchtelemente aus Halbleiter-Lichtquellen (LED) mit entsprechender spektraler Verteilung, welche in der in Fig. 2 dargestellten Art auf einem in Zonen unterteilten Träger montiert sind.

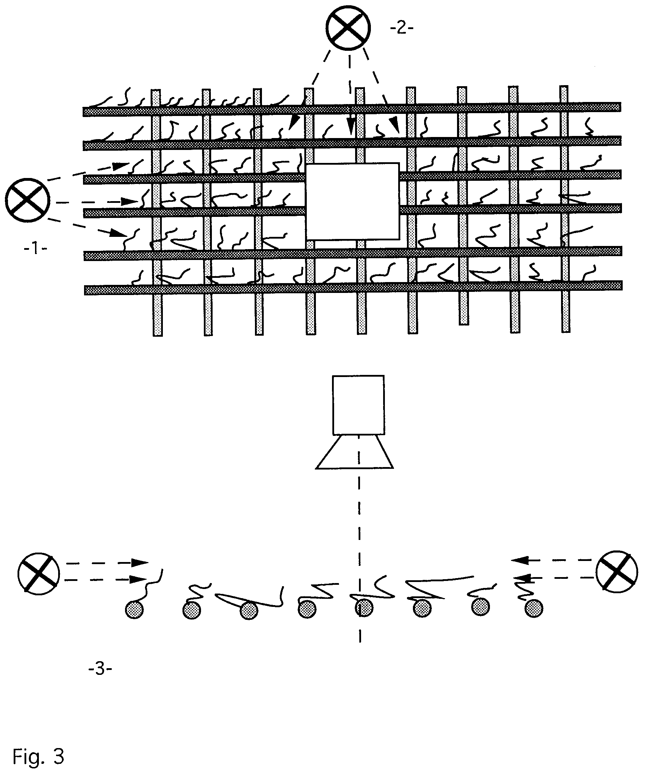

Beispielsweise sollen die Parameter "Richtung der Kette". "Richtung der Schußfäden" und "Faserigkeit" gleichzeitig bestimmt werden. Hierzu werden drei Bilder mit jeweils folgender Beleuchtungsart nach Fig. 3 aufgenommen:
1. zur Gewinnung eines Bildes, in dem die Kettenfäden besonders kontrastreich hervortreten, werden die Zonen eingeschaltet, welche senkrecht zur Kette liegen (Fig. 3 Detail 1),
2. zur Gewinnung eines Bildes, in dem die Schußfäden besonders kontrastreich hervortreten, werden die Zonen eingeschaltet, welche senkrecht zu den Schußfäden liegen (Fig. 3, Detail 2),
3. zur Gewinnung eines kontrastreichen Bildes, welches die Fasern besonders kontrastreich erscheinen läßt, wird in einem extrem großen Winkel zur Oberflächennormalen nach Fig. 3, Detail 3 beleuchtet.

Die Auswertung dieser Bilder mit den Verfahren der Bildverarbeitung zur Gewinnung der Richtung von Kette und Schuß bzw. zur Gewinnung eines Maßes für die Faserigkeit ist dem Fachmann der Bildverarbeitung bekannt und braucht hier nicht weiter verdeutlicht zu werden. Exemplarisch wird z. B. die Richtung der Kette und der Schußfäden im jeweiligen Bild durch Anwendung der Hough-Transformation auf das ermittelte Grauwertbild erreicht (siehe z. B. Haberäcker, Digitale Bildverarbeitung, Hanser Verlag München). Als Maß für die Faserigkeit kann die Energie im zweidimensionalen Fourierspektrum, begrenzt auf hohe Ortfrequenzen, verwendet werden.

Ein wesentlicher Vorteil der Erfindung besteht darin, mit einer statischen, mechanisch nicht bewegten Anordnung von bildgebendem Sensor und Beleuchtungsquelle durch elektronische Umschaltung der Beleuchtung und Umschaltung eines entsprechenden Programms, welches das für die jeweilige Meßart bestimmte Verfahren berechnet, eine große Zahl von Qualitätsmerkmalen ermitteln zu können.

Für die Berechnung des Flächengewichts wird die Beleuchtung so eingestellt, daß die Poren zwischen Kette/Schuß bzw. Maschen/Stäbchen besonders gut sichtbar sind. Das Flächengewicht ist dann proportional zu dem Verhältnis von hellen zu dunklen Stellen im Bild. Die Anzahl der Ketten/Schußfäden bzw. Maschen/Stäbchen pro Längeneinheit ist ein indirektes Maß für das Flächengewicht.

Bei vielen bedruckten Textilien ist es wichtig, die optische Reflexion zu überprüfen, da lokale Inhomogenitäten des Reflexionskoeffizienten sich als fleckenartige Störungen äußern. Hierzu werden diejenigen Beleuchtungsquellen eingeschaltet, welche der Farbe entsprechen, bei denen das Material besonders gut reflektiert. Durch die Beleuchtung mit einer Farbe, welche weit entfernt von der reflektierenden Farbe liegt, kann erfindungsgemäß ein zur Normierung auf einen Dunkelwert bestimmtes Bild gewonnen werden.

Ein bisher noch ungenügend gelöstes Problem war die Identifikation einer nicht markierten Stoffpartie. Erfindungsgemäß wird aus den vorgeschlagenen Qualitätsparametern ein Merkmalsvektor gebildet. Dieser Merkmalsvektor wird mit einer Bibliothek vorher gelernter Referenzvektoren verglichen und nach den bekannten Verfahren der Klassifikation der Typ der unbekannten Partie gleichgesetzt mit dem Typ derjenigen Referenz, zu welcher die größte Ähnlichkeit besteht. Bekannte Ähnlichkeitsmaße sind der Korrelationskoeffizient, der komplementäre Wert zur euklidischen Distanz usw.

Eine weitere Möglichkeit der Beleuchtung besteht im Rahmen der Erfindung in der Verwendung parallelstrahliger Lichtbündel zur homogenen und gleichgerichteten Beleuchtung strukturierter Oberflächen mit unterschiedlich parallelen Einstrahlwinkeln über Holografische Elemente.

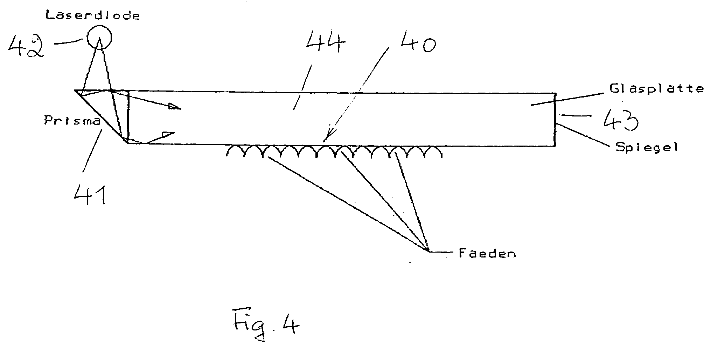

Bei streifender Beleuchtung der Bahnoberfläche zur Erfassung mikroskopischer Teilstrukturen, wie Faserigkeit, Haarigkeit oder dergl. können mehrere Vorzugsrichtungen für die Beleuchtung unter Ausnutzung unterschiedlichster Brechungsindizes (Luft und Material) gewählt werden. Auf der der Beleuchtungseinrichtung gegenüber liegenden Seite der Warenbahn können die Strahlung refklektierende Spiegel vorgesehen sein. Eine praktische Ausführungsform zeigt Fig. 4. Hier erfolgt die Lichteinkoppelung zur streifenden Beleuchtung der Textiloberfläche 40 mit Hilfe eines Prismas 41 von einer Laserdiode 42 aus. Der reflektierende Spiegel ist mit 43 bezeichnet, eine das Licht führende Glasplatte mit 44.

Soll eine strukturierte Oberfläche (Textilien, Siebe etc.) ohne genaue Kenntnis der Vorzugsrichtung der Textur einer gerichteten Ausleuchtung unterworfen werden, empfiehlt sich dazu eine segmentierte Ringbeleuchtung, wobei die einzelnen Beleuchtungssegmente so angesteuert werden können, daß entweder ein Segment zeitlich nacheinander die Oberfläche aus unterschiedlichen Richtungen beleuchtet oder mehrere Segmente mit einem Winkel zwischen den Segmenten die Oberfläche, zeitlich nacheinander, umlaufend aus unterschiedlichen Richtungen beleuchtet. Die anwendungsbezogene Optimierung ergibt dann die Auswahl des jeweils günstigsten Signalkontrastes.

## Patentansprüche

1. Verfahren zur berührungslosen optischen Messung von qualitätsbestimmenden Parametern textiler Oberflächen mit Hilfe bildgebender Sensoren, dadurch gekennzeichnet, daß die textile Oberfläche ausschnittsweise mit einem bildgebenden Sensor erfaßt wird, daß dieser Ausschnitt durch eine Beleuchtungsquelle mit einer steuerbaren Verteilung der drei-dimensionalen Beleuchtungsrichtung und/oder der Beleuchtungszeit und/oder der spektralen Verteilung beleuchtet wird, wobei in Abhängigkeit von der textilen Oberfläche und der durchzuführenden Meßart die jeweils beste Beleuchtungsart eingestellt wird, daß das Signal des bildgebenden Sensors digitalisiert und in einen oder mehrere Bildspeicher abgelegt wird, und daß mit einer Recheneinheit anhand des oder der abgespeicherten Bilder mit einem jeweils auf die interessende Messung abgestimmten Verfahren der digitalen Signal- oder Bildverarbeitung und Mustererkennung alle oder eine Teilmenge folgender qualitätsbestimmender Parameter ermittelt werden:
1. die Anzahl von Maschen und Stäbchen pro Längeneinheit,
2. die Anzahl von Ketten und Schußfäden bei Webware,
3. die Ausrichtung der Maschen und Stäbchen,
4. die Ausrichtung der Kette und der Schußfäden,
5. das Flächengewicht,
6. die lokale Homogenität bei Maschen- und Webware,
7. die Faserigkeit der textilen Oberfläche
8. das optische Reflexionsverhalten bei verschiedenen Wellenlängen,
9. das optische Transmissionsverhalten bei verschiedenen Wellenlängen,
10. die automatische Identifikation bei verschiedenen Wellenlängen,
11. Die Konsistenz der visuellen Eigenschaften der Oberfläche.

2. Verfahren nach Anspruch 1, dadurch gekennzeichnet, daß aus den ermittelten Qualitätsmerkmalen Signale zur Steuerung des Produktionsprozesses abgeleitet werden.

3. Verfahren nach Anspruch 1, dadurch gekennzeichnet, daß die Beleuchtungseinheit in zwei Teile gegliedert ist, von denen der eine Teil die Oberfläche im Auflicht und der andere Teil die Oberfläche im Durchlicht beleuchtet.

4. Verfahren nach Anspruch 1, dadurch gekennzeichnet, daß zur Bestimmung des Reflexionsverhaltens einer textilen Oberfläche nur diejenigen Leuchtelemente eingeschaltet werden, deren Spektrum mit dem Soll-Spektrum der Reflexion möglichst übereinstimmt.

5. Verfahren nach Anspruch 4, dadurch gekennzeichnet, daß zur Normierung des Reflexionskoeffizienten die textile Oberfläche mit einem Leuchtelement beleuchtet wird, dessen Spektrum sich möglichst von dem der Reflexion unterscheidet und daß dieser Reflexionswert zur Normierung verwendet wird.

6. Verfahren nach Anspruch 4 oder 5, dadurch gekennzeichnet, daß sequentiell die Oberfläche mit Beleuchtungselementen unterschiedlicher Wellenlänge beleuchtet wird und damit die Abhängigkeit der Reflexion von der Wellenlänge bestimmt wird.

7. Verfahren nach Anspruch 1, dadurch gekennzeichnet, daß alle oder eine Untermenge der qualitätsbestimmenden Parameter zu einem Merkmalsvektor zusammengefaßt werden, daß durch Vergleich dieses Merkmalsvektors mit einer Referenzbibliothek vorher gelernter Vektoren die Klasse bestimmt wird, zu der die betreffende Stoffpartie gehört.

8. Anordnung zur Durchführung des Verfahrens nach Anspruch 1, dadurch gekennzeichnet, daß die Beleuchtungseinrichtung aus einer im Raum gekrümmten Anordnung von zu Zonen zusammengefaßten Anordnungen von punktförmigen Leuchtelementen unterschiedlicher Wellenlänge besteht, welche jeweils einzeln, in Gruppen, zeitgleich oder zeitlich versetzt und synchron zur Bildaufnahme steuerbar sind.

9. Anordnung nach Anspruch 2, dadurch gekennzeichnet, daß die Leuchtelemente aus Halbleiter-Leuchtioden bestehen.
